# EUROPEAN PATENT SPECIFICATION

(11) **EP 2 065 705 B1**
(45) Date of publication and mention of the grant of the patent: **27.07.2011**
(21) Application number: 08020499.3
(22) Date of filing: 26.11.2008
(51) Int. Cl.: G01N 33/532, A61K 47/48

(54) **Stabilization of conjugates comprising a thiourea linker**
Stabilisierung von Konjugaten mit einem Thiourea-Binder
Stabilisation de conjugués comprenant un agent de liaison de thiourée

(30) Priority: 30.11.2007 EP 07023206
(43) Date of publication of application: 03.06.2009
(73) Proprietor: Roche Diagnostics GmbH, 68305 Mannheim (DE); F.Hoffmann-La Roche AG, 4070 Basel (CH)
(72) Inventor: Blind, Rene, 82396 Paehl (DE); Fink, Gerhard, 83673 Bichl (DE); Hoess, Eva, 82061 Neuried (DE); Kytzia, Hans-Joachim, 79227 Schallstadt (DE)

(56) References cited:
- EP-A- 1 795 607
- WO-A-2006/032441
- AU-A4- 2006 100 657
- JP-A- 57 018 605
- JP-A- 58 055 417
- US-A1- 2003 204 113
- ACHILEFU SAMUEL ET AL: "Synthesis, in vitro receptor binding, and in vivo evaluation of fluorescein and carbocyanine peptide-based optical contrast agents." JOURNAL OF MEDICINAL CHEMISTRY 9 MAY 2002, vol. 45, no. 10, 9 May 2002 (2002-05-09), pages 2003-2015, XP002476818 ISSN: 0022-2623

## Description

### Background of the Invention

The present invention discloses a composition containing a conjugate comprising two moieties covalently linked by a thiourea linker and thiourea or a derivative thereof in free form. It also relates to a method for stabilizing a conjugate comprising two moieties covalently linked by a thiourea linker is described the method comprising the steps of providing the conjugate, adding thereto thiourea or a derivative thereof in free form and thereby stabilizing the conjugate and to the use of a composition containing a conjugate comprising two moieties covalently linked by a thiourea linker and thiourea or a derivative thereof in free form.

A large variety of homo- and heterobifunctional linkers are available to the chemist to covalently link two moieties of interest via such linker to each other. Thiourea is a short and convenient to use homobifuntional linker.

Thiourea as a linker may always be given a try once homobifunctional linkers are evaluated for their applicability to link two moieties together in order to obtain a conjugate of these two moieties that are covalently linked via the thiourea linker to each other.

In some instances the use of thiourea brings about additional desired and advantageous effects. Such advantageous properties of a thiourea linker have for example been described in US 6,586,460. A thiourea linker for example, may also represent the linker bringing about the appropriate distance between two moieties of interest. In certain other instances the reactive groups present in the moieties to be linked to each other warrant the use of thiourea. Or, as the case may be, a first intermediate product is synthesized comprising one of the moieties of interest and thiourea already attached thereto, thereby allowing the coupling to a second moiety of interest via the free amino group of thiourea still available for said coupling.

Samuel Achilefu et al. (J. Med. Chem. 45 (2002) 2003-2015) report on a conjugate between fluorescein and a carbocyanine peptide comprising thiourea as a linker.

WO 2006/032441 describes e.g. conjugates between fluoresceinisothiocyante and cellobiose or mannose, respectively.

Often a thiourea linker will simply represent the most convenient homobifunctional linker of choice.

A thiourea linker, if a conjugate comprising such linker is stored with care, will usually be rather stable. In cases, however, where storage conditions can not be extensively controlled, for example during transport in a non-refrigerated transport chain, a conjugate comprising a thiourea linker may deteriorate.

Negative effects of thermal stress may also become evident and require compensatory measures under onboard stress conditions. If for example a conjugate comprising two moieties e.g. an analyte and a label covalently linked by a thiourea linker is used as a so-called tracer material in an immunoassay, such reagent should be stable as long as possible both during transport as well as on board of an analyzer.

Obviously, it would be quite advantageous, if deterioration of a conjugate comprising two moieties covalently linked by a thiourea linker could be prevented and the conjugate thereby protected or stabilized. By such stabilization the deterioration of the conjugate would be reduced. This for example could lead to an improved stability during shipment of such a conjugate or under long term storage conditions.

It has now been found and could be established that thiourea in free form or an appropriate derivative thereof, if added to a conjugate comprising two moieties covalently linked by a thiourea linker is very effective in reducing deterioration of such conjugate.

The compostions and methods described in the present invention appear to be appropriate to improve both the stability of a conjugate comprising two moieties covalently linked by a thiourea linker both under transport conditions as well as onboard of an analyzer.

### Summary of the Invention

The present invention discloses a composition containing a conjugate comprising two moieties covalently linked by a thiourea linker and thiourea or a derivative thereof in free form.

A method for stabilizing a conjugate comprising two moieties covalently linked by a thiourea linker is described the method comprising the steps of providing the conjugate, adding thereto thiourea or a derivative thereof in free form and thereby stabilizing the conjugate.

Also disclosed is the broad applicability and use of thiourea or of an appropriate derivative thereof as a stabilizer for a conjugate comprising two moieties covalently linked by a thiourea linker.

### Detailed Description of the Invention

In a first embodiment the present invention relates to a composition containing a purified conjugate comprising two moieties covalently linked by a thiourea linker and thiourea or a derivative thereof as defined in the claims in free form.

As indicated above the inventors of the present invention have found that thiourea in free from can stabilize a conjugate comprising a thio urea linkage, this is especially true if the conjugate in need of stabilization is atored in liquid form. As the skilled artisan will appreciate, such stabilizing effect does not necessarily require the use free thiourea, because derivatives of thiourea exhibiting similar chemical properties with respect to stabilization may also be used. The skilled artisan can easily select appropriate thiourea derivatives. Appropriate but non-limiting examples of thiourea derivatives are Dithiobiurea, N-Acetylthiourea, 2-Imidazolinethione, N,N'-Dimethylthiourea, 1-Methyl-2-thiourea and 1,1,3,3,-Tetramethyl-2-thiourea. Preferably the thiourea derivative will be an alkylated form of thiourea, wherein one, two, three or all four hydrogen atoms of thiourea are substituted by lower alkyl, i.e. with alkyl of six or less carbon atoms. Preferably such alkyl will be either/or methyl and/or ethyl. Other preferred substituents at one or both the nitrogens of thiourea encompass acetyl, C1-C6 alkenyl and thiourea. In a further preferred thiourea derivative an alkyl or alkylen bridge between the two nitrogen atoms having two or three C-atoms is present. As the skilled artisan will appreciate, it is also possible to use a mixture of both free thiourea and a thiourea derivative or to use a mixture of different thiourea derivative in order to stabilize a conjugate comprising a thiourea linker.

The term "purified" relates to the level of the desired conjugate as compared to the level of reaction intermediates and reaction by-products. A purified conjugate is a preparation of such conjugate that consists to at least 75 %, or 80 %, or 85 %, or 90 %, or 95 %, 96 %, 97%, 98% or to at least 99 % of the conjugate of interest. Preferred levels of purity are at least 90 %, at least 95 % or at least 98 %, respectively.

The moieties covalently linked by a thiourea bridge may be any moiety of interest in organic chemistry or in biochemistry. Preferably at least one moiety will have a molecular weight of 100 Dalton or more, or also preferred of 150 or 200 Dalton or more. Also preferred both moieties will have molecular weight of 100 Dalton or more, or also preferred of 150 or 200 Dalton or more. Preferably at least one of the two moieties linked by the thiourea linker will have a molecular weight of 20 000 Dalton or less, also preferred of 10 000 Dalton or less or of 5000 Dalton or less.

Preferably the conjugate comprised in a composition according to the present invention is of formula I.

R1,R2-N-CS-N-R3,R4 Formula I:

wherein R1 represents a first moiety that is capable of binding to a binding partner, wherein R2 and R3 independently represent H, C1-C6 alkyl, C1-C6 alkenyl, or acetyl, or wherein R2 and R3 form a C2 or a C3 alkyl or alkylen bridge between the two nitrogen atoms, and wherein R4 represents a label.

Preferably the composition of the present invention comprises at least a first moiety that is capable of binding to a binding partner.

Preferably the binding partner is a member of a specific binding pair. Well-known, and suitable binding pairs in biology, biochemistry or immunochemistry are a hapten or an antigen capable of binding to an antibody, biotin or biotin analogues such as aminobiotin, iminobiotin or desthiobiotin capable of binding to avidin or streptavidin, sugar, capable of binding to lectin, a nucleic acid or a nucleic acid analogue capable of binding to a complementary nucleic acid, a receptor capable of binding to a ligand, e.g., a steroid hormone receptor capable of binding a steroid hormone. These binding pairs may also be referred to as bioaffine binding pairs. Preferred binding pair members are a hapten for an antigen and an antibody binding to this hapten or to this antigen, respectively. Also preferred is a peptidic binding partner that is e.g. obtainable by phage display (see e.g., Allen et al., TIBS 20 (1995) 511-516).

In a preferred embodiment the binding partner to which the at least first moiety is capable of binding is an antibody and the first moiety represents an analyte or an analyte derivative.

A binding partner has at least an affinity of 10⁷L/mol for its corresponding target molecule, i.e. the second partner of a binding pair. The binding partner preferably has an affinity of 10⁸L/mol or even more preferred of 10⁹L/mol for its target molecule.

Also preferred, the binding partner specifically binds to the target molecule of interest. As the skilled artisan will appreciate the term specific frequently is used to additionally indicate that biomolecules other than the target molecule present in a sample do not significantly bind to the specific binding agent. Preferably the level of binding to a biomolecule other than the target molecule results in a binding affinity which is only 10% or less, more preferably only 5% or less of the affinity for the target molecule. A most preferred specific binding partner will fulfill both the above minimum criteria for affinity as well as for specificity.

Preferably the at least one first moiety that forms part of a conjugate to be stabilized by a method or in a composition according to the present invention is a low molecular weight analyte or an immunologically equivalent derivative thereof

Preferably such low molecular weight analyte has a molecular weight of 2000 Dalton or less. The term immunologically equivalent derivative is used to indicate that the at least first moiety must not necessarily be absolutely identical to the low molecular weight analyte of interest. Rather it frequently is sufficient to use a closely related structure or derivative of said analyte for forming the conjugate as discussed above. The analyte derivative will suffice as an equivalent or substitute for the target analyte as long as it binds in the same manner to the binding partner used in the detection of said analyte of interest. Preferably the first moiety is capable of binding to an antibody.

Preferred low molecular analytes are therapeutic drugs and drugs of abuse. The therapeutic drug is preferably selected from the group consisting aminoglycosides, like amikazine, gentamicin, and trobramicin, carbamazepine, lidocaine, procainamid, barbiturates like phenobarbiturate and secobarbiturate, phenytoin, primidone, quinidine, thyroxine, T3, theophylline, valproic acid, vancomycin, and physiological metabolites or derivatives of all of them.

The drug of abuse is preferably selected from the group consisting of amphetamine, cocaine and cocaine metabolites like benzoylecgnonine, methamphetamine, opiate and opiate derivatives, cannabinoids like tetrahydrocannabinol, and phencyclidine. A further preferred target analyte is folate, especially the so-called total folate as comprised in both the blood plasma and in the red blood cells.

Preferred target analytes are immunosuppressive drugs. The immunosuppressive drug is preferably selected from the group consisting of cyclosporine (CsA), mycophenolate mofetil (MMF), rapamycin (RAPA also known as sirolimus), tacrolimus (FK-506) azathioprine (AZA), and methylprednisolone (MP).

In a further preferred embodiment the conjugate contained in the composition of the present invention comprises a label. A label is a directly or an indirectly detectable substance, respectively.

The label according to the present invention may be a directly detectable label or an indirectly detectable label. Both these types of labels are well-known to the skilled artisan and the artisan will select the appropriate label according to his needs.

Well-known and preferred labels that are directly detectable are, luminescent groups, for example chemiluminescent groups like acridinium esters or dioxetanes, or fluorescent dyes e.g. fluorescein, coumarin, rhodamine, oxazine, resorufin, cyanine and derivatives thereof. Other preferred examples of directly detectable labeling groups are luminescent metal complexes such as ruthenium or europium complexes, enzymes for example enzymes used in ELISA methods or parts of enzymes as for example used in CEDIA (Cloned Enzyme Donor Immunoassay, e.g. EP-A 0 061 888), and radioisotopes.

Indirectly detectable labels are groups that are capable of binding to a specific binding partner that carries a directly detectable label as described in the previous paragraph.

Preferred indirectly detectable labels are low molecular weight binding partners of high affinity binding pairs like haptens capable of binding to an antibody, biotin capable of binding to avidin or streptavidin or nucleic acids capable of binding to complementary nucleic acids. Haptens are small molecules with a molecular weight of less than 2 kDa, preferably less than 1.5 kDa and most preferred less than 1 kDa. A hapten itself is not immunogenic. However, when conjugated to an appropriate carrier molecule, such conjugate may be used to render the hapten immunogenic and thus to generate an appropriate immune response. Polyclonal as well as monoclonal antibodies against haptens of various kinds are well-known in the art. Well-known and preferred haptens are for example biotin, di-nitro-phenyl-phosphate, steroidal hormones, many low molecular weight drugs, digoxin and digoxigenin.

In order to achieve a reasonable stabilization of a conjugate comprising a thiourea linker, the skilled artisan will determine which molecular ratio of conjugate to free thiourea is best suited to achieve the desired effect. In a preferred embodiment the composition according to the present invention will contain thiourea or a derivative thereof in free form in an at least equimolar concentration as compared to the thiourea linker comprised in the conjugate. Also preferred the composition according to the present invention will contain thiourea or a derivative thereof in free form in an at least a 10-molar excess as compared to the thiourea linker comprised in the conjugate.

Preferably the composition of the present invention will comprise the thiourea or a derivative thereof in an about 1000-fold molar excess as compared to the concentration of the thiourea-containing conjugate requiring stabilization/protection.

High concentrations of thiourea or a derivative thereof do not appear to have a negative impact on the conjugate to be stabilized. The skilled artisan will have no problem to select the highest concentration of thiourea or a derivative thereof for his intended use. Preferably the composition of the present invention will comprise the thiourea or a derivative thereof in an about 10⁵-fold molar excess or less as compared to the concentration of the thiourea-containing conjugate requiring stabilization/protection. Also preferred the molar excess will be 10⁴-fold or less or 5000-fold or less as compared to the concentration of the thiourea-containing conjugate requiring stabilization/protection.

In a preferred embodiment the present invention relates to a method for stabilizing a conjugate comprising two moieties covalently linked by a thiourea linker, the method comprising: providing the conjugate, adding thereto thiourea or a derivative thereof as defined in the claims in free form and thereby stabilizing the conjugate.

Usually the conjugate comprising two moieties covalently linked by a thiourea linker is chemically synthesized and the desired conjugate is purified by appropriate means, like chromatography. The conjugate usually may be stored under appropriate storage conditions, preferably frozen until needed to formulate a liquid composition comprising the conjugate. The liquid, preferably a buffer, receiving the conjugate, may already contain the free thiourea or a derivative therof or these stabilizers may be added into the liquid together or after the addition of the conjugate.

As the skilled artisan will appreciate, the method for stabilization of a conjugate comprising a thiourea linker that covalently links two moieties may be practiced with any of the preferred conjugates and moieties, respectively, as described herein further above.

Preferably the method will be practiced with a conjugate comprising a thiourea linker that covalently links two moieties and having at least a first moiety that is capable of binding to a binding partner.

Also preferred the method can be applied to stabilize a conjugate comprising a thiourea linker that covalently links two moieties and having at least a second moiety that is a labeling group.

Also preferred the method can be applied to stabilize a conjugate comprising comprising a thiourea linker that covalently links two moieties and having at least a first moiety that is capable of binding to a binding partner and at least a second moiety that is a labeling group.

Preferably the method to stabilize the conjugate comprising a thiourea linker that covalently links two moieties is practiced by adding thiourea in free form or a thiourea derivative in free form to a final concentration that is at least an equimolar concentration and in at most a 10⁵-fold molar excess as compared to the thiourea linker comprised in the conjugate.

A further referred embodiment relates to the use of thiourea or a derivative thereof as defined in the claims as a stabilizer for a conjugate comprising two moieties covalently linked by a thiourea linker.

The following examples and figures are provided to aid the understanding of the present invention, the true scope of which is set forth in the appended claims.

### Description of the Figures

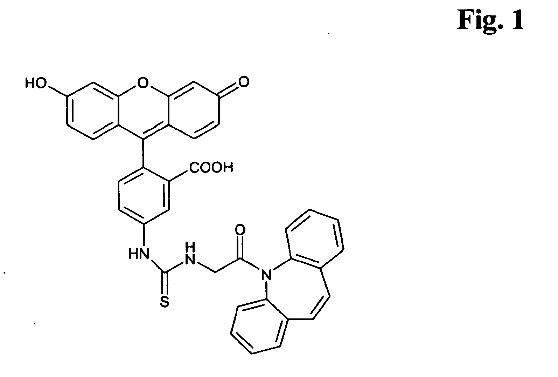

- **Figure 1**: **Structure of a carbamazepine-fluorescein conjugate** The conjugate depicted comprises a moiety that is capable of binding to a binding partner (the carbamazepine that can be bound by an antibody thereto) and a label (fluorescein) which are linked to each other by thiourea.

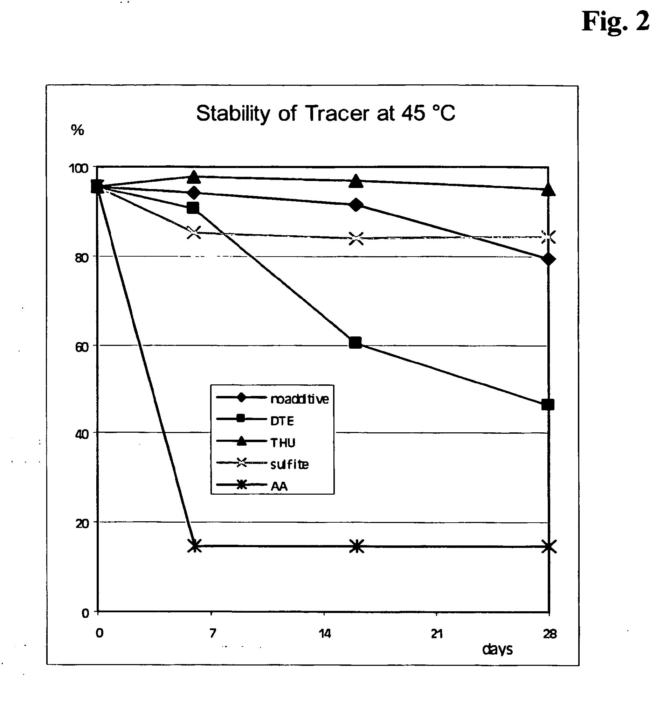

- **Figure 2**: **Stress stability of the carbamazepine tracer** Depicted is the tracer stability at 45°C for tracer preparations without additive (no additive), or tracer preparations comprising ascorbic acid (AA), thiourea (THU), sodium sulfite (Na₂SO₃) (sulfite) and 1,4-dithiothreitol (DTE), respectively. The time points shown are 0, 1, 2, 3, and 4 weeks, respectively.

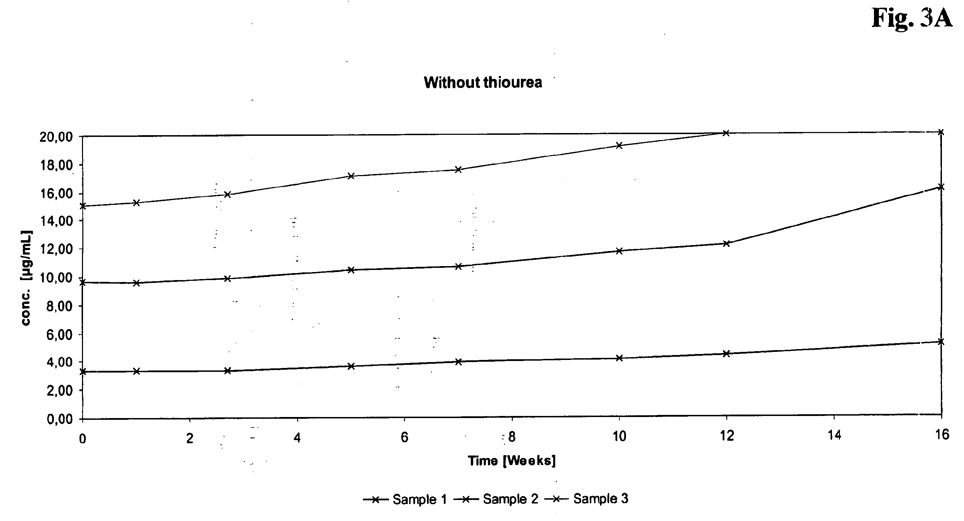

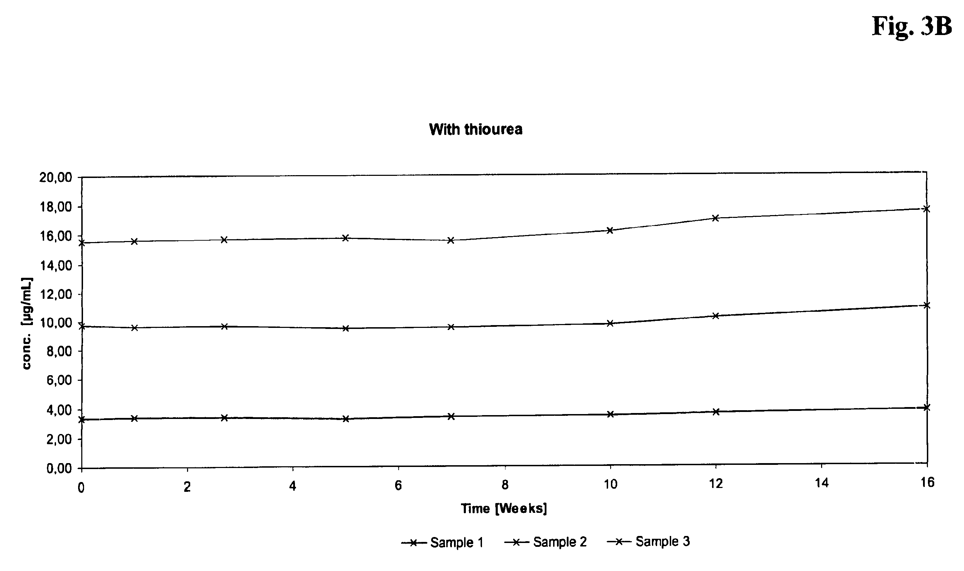

- **Figure 3**: **Effect of onboard stability/instability of a tracer conjugate on the read-out for control samples** Control samples comprising three different levels of carbamazepine have been measured with a tracer preparation left on the analyzer for up to 12 weeks. The calculated concentrations for these controls are given dependent on the onboard storage time. Higher values translate to a deterioration of tracer. Data for two preparations of tracer conjugate (cf. Fig: 1) without (Figure 3A) and with thiourea (Figure 3B) as a stabilizer, respectively, are shown.

### Example 1

### Effect of various antioxidants on the stability of a thiourea containing conjugate

The conjugate depicted in Fig: 1, comprising carbamazepine as a moiety that is capable of binding to a binding partner, fluorescein as a label and a thiourea linker is used as a model conjugate to demonstrate both the stability problems that may be encountered as well as the way to stabilize such conjugate.

The stability of the carbamazepine-fluorescein conjugate in buffer containing different anti-oxidants was investigated. Various antioxidants have been evaluated for their effect on stabilizing the conjugate as used in the tracer reagent. Ascorbic acid (AA), thiourea (THU), sodium sulfite (Na₂SO₃) and 1,4-dithiothreit (DTE) have been added to the tracer reagent of the carbamazepine assay in a final concentration of 20 mmol/L. 320 µl 50 mM Tris buffer at pH 7.5 with or without 20 mM antioxidants were mixed with 40 µl DMF, and 40 µg conjugate dissolved in these 40 µl of DMF.

Aliquots of each of the resulting reagent mixtures have been stored at 4°C whereas other aliquots have been subjected to thermal stress by storage at 45°C. These aliquots have been compared to each other as well as to a tracer preparation not containing an antioxidant. Tracer stability was analyzed after one, two, three and four weeks, respectively.

The samples were analyzed by HPLC using a reversed phase column (Zorbax C3) and an acidic water/acetonitril gradient at 60 °C every week. The stability of the conjugate was calculated from the peaks obtained in the HPLC analysis.

As obvious from Figure 2, the stabilizing effect on the carbamazepine-fluorescein conjugate, i.e. a conjugate between two moieties comprising a thiourea linker, appears not to be due to a mere antioxidant effect, since only thiourea provides for stabilization at 45°C, whereas antioxidants like ascorbic acid or dithiothreitol even had a negative impact on conjugate stability.

### Example 2

### Compatibility of thiourea with immunological reagents

Carbamazepine can e.g. be measured with the commercially available Carbamazepine assay kit from Roche Diagnostics (Order-No. 20737828). In this assay a carbamazepine-fluorescein conjugate is used as a tracer reagent.

In brief the carbamazepine assay commercially available from Roche works as follows. A standard or calibration curve is established by reacting different amounts of carbamazepine with the conjugate of Fig: 1 and with an antibody to carbamazepine, in a similar manner an aliquot of the sample to be analyzed is mixed with a reagent comprising the carbamazepine-fluorescein conjugate and a reagent comprising an antibody to carbamazepine. Carbamzepine in the sample (or standard) and the conjugate compete for binding to the antibody. The antibody-carbamazepine-fluorecein complex is specifically detected. The more carbamezepine is in a sample the less signal will be generated for that sample. The sample signal is read of the calibration curve and thereby translated into the concentration of carbamazepine in the sample. The full procedural details of the commercially available assay can be derived from the package insert for Roche Diagnostics Cat.-No. 20737828 and need not to be reproduced here. In case of questions relating to details of the measurement, specific reference is hereby made to the package insert.

The tracer comprising the carbamazepine-fluorescein conjugate, i.e. a conjugate wherein the moieties carbamazepine and fluorescein are linked by thiourea was used. For evaluation of the effects of thiourea on the immunological reagents of the commercial carbamazepine assay, the measurement was performed according to the manufacturer's instructions. The tracer reagent for this experiment was prepared with and without thiourea (20 mM), respectively.

As can be seen from Table 1, the addition of thiourea (THU) to a tracer reagent mixture set up in analogy to the tracer as used in the commercial product, does not interfere with the measurement of carbamazepine, because comparable signals are measured for a tracer with and a tracer without thiourea.

**Table 1:**

| **Signal output with and without thiourea as a stailizer** | | | |
|---|---|---|---|
| **Standard** | **carbamazepine in µg/mL** | **rate (arbitrary units) without THU** | **rate (arbitrary units) with THU** |
| Std-F | 20 | 125,91 | 126,54 |
| Std-E | 10 | 148,96 | 150,65 |
| Std-D | 5 | 180,09 | 181,52 |
| Std-C | 2,5 | 213,43 | 215,87 |
| Std-B | 1,25 | 240,25 | 242,35 |
| Std-A | 0 | 268,88 | 269,48 |

### Example 3

### Effect of thiourea on thermal stress stability of a thiourea linked conjugate

Long term storage conditions at refrigerated temperature as well as transport temperature stress conditions are frequently assessed by short term thermal stress models. The tracer reagent for this experiment was prepared with and without thiourea (20 mM), respectively, and the measurement was performed according to the manufacturer's instructions. The stability of the solution containing the carbamazepine-fluorescein conjugate in the presence or in the absence of free thiourea (20 mM) as a stabilizer was assessed after three weeks storage at 45°C and compared to a tracer solution not containing a stabilizer analysed at day zero.

As can be seen from Table 2, the addition of thiourea to a tracer reagent mixture set up in analogy to the tracer as used in the commercial product, does stabilize such reagent mixture even if stored under thermal stress conditions, i.e. for three weeks at 45°C.

**Table 2:**

| **Effect of free thiourea on tracer stability under thermal stress** | | | |
|---|---|---|---|
| **Rate** | **day 0** | **3 weeks@45°C** | **3 weeks@45°C** |
| **Standard** | **without thiourea** | **without thiourea** | **with thiourea** |
| Std-F | 127,42 | 119,78 | 125,74 |
| Std-E | 151,18 | 141,23 | 148,57 |
| Std-D | 180,14 | 168,25 | 178,86 |
| Std-C | 213,34 | 201,40 | 210,42 |
| Std-B | 242,59 | 227,37 | 238,27 |
| Std-A | 277,80 | 262,60 | 272,62 |

As mentioned above, the calibrator solutions, i.e. those solutions used to establish a calibration curve from which absorbence values and corresponding concentrations can be read of and calculated, also contain the conjugate of Fig: 1.

### Example 4

### Effect of thiourea concentration on stability of a thiourea linked conjugate

Three different concentrations of thiourea, i.e. 10 mM, 20 mM and 30mM, respectively have been assessed for their stabilizing effects on four different preparations of carbamazepine-fluorescein conjugates. The measurement was performed as described above. Stability of the tracer conjugate was assessed by reading of the signals for the highest calibrator as comprised in the commercially available carbamazepine assay after storage at 45°C for two weeks in comparison to the signal at day zero.

As is obvious from Table 3, all the three concentrations of thiourea tested appear to stabilize a carbamazepine-fluorescein conjugate. Whereas the degree in signal loss without stabilizer shows some variability, the stabilizing effect of free thiourea appears to eliminate instability independent of the basic stability properties (i.e. without addition of thiourea) of such a carbamazepine-fluorescein conjugate.

**Table 3:**

| **Signal loss for the highest calibrator** | | | | |
|---|---|---|---|---|
| **Cal A [Δmp]** | **Lot 1** | **Lot 2** | **Lot 3** | **Lot 4** |
| 0 mmol/L | 18,10 | 11,89 | 7,94 | 8,15 |
| 10 mmol/L | 4,01 | 3,41 | 4,75 | 4,18 |
| 20 mmol/L | 4,94 | 5,60 | 3,48 | 3,45 |
| 30 mmol/L | 4,82 | 4,20 | 3,81 | 4,37 |

### Example 5

### Effect of thiourea-mediated tracer stabilization on carbamazepine recovery

A rather instable lot of a carbamazepine-fluorescein conjugate was used to set up a tracer solution with and without thiourea as a stabilizer and the onboard stability of such a reagent has been investigated over a period of 16 weeks. Tracer stability was indirectly assessed by a quantitative measurement of three different sample containing 3.33, 9.66 and 15.07 µg/ml of carbamazepine, respectively.

As can be easily told from a comparison of Figures 3a and 3b, the addition of thiourea in free from does also stabilize the tracer conjugate onboard of the analyzer. Free thiourea if added to a conjugate comprising a thiourea linker appears to improve the conjugate stability under various requirements like short thermal stress conditions or onboard of an automatica analyzer.

### Example 6

### Assessment of various thiourea derivatives

Different thiourea derivatives were added to the carbamazepine tracer reagent in a final concentration of 20 mmolar. The calibrator signals were measured at day 0 and after 15 days at 4°C and at 35°C, respectively. All values in Tables 4 and 5, respectively are given in % of the corresponding value for day 0. In the column labeled reference thiourea has been used as a stabilizer, whereas in CARB 1 Dithiobiurea, in CARB2 N-Acetylthiourea, in CARB3 2-Imidazolinethione, in CARB4 N,N'-Dimethyl-thiourea, in CARB5 1-Methyl-2-thiourea and in CARB6 1,1,3,3-Tetramethyl-2-thiourea, respectively, have been used as a stabilizer.

**Table 4:**

| **Recovery of the calibrator signals after storage 15 days at 4°C:** | | | | | | | |
|---|---|---|---|---|---|---|---|
| **Standard** | **Reference** | **CARB1** | **CARB2** | **CARB3** | **CARB4** | **CARB5** | **CARB6** |
| Std-F | 99% | 100% | 100% | 100% | 100% | 100% | 100% |
| Std-E | 100% | 100% | 101% | 100% | 100% | 101% | 100% |
| Std-D | 99% | 100% | 101% | 100% | 100% | 101% | 100% |
| Std-C | 99% | 100% | 100% | 100% | 100% | 101% | 99% |
| Std-B | 100% | 100% | 101% | 100% | 100% | 101% | 100% |
| Std-A | 100% | 100% | 100% | 100% | 100% | 100% | 99% |

**The recovery was fine with all used thiourea derivatives.**

**Table 5:**

| **Recovery of the calibrator signals after storage 15 days at 35 °C:** | | | | | | | |
|---|---|---|---|---|---|---|---|
| **Standard** | **Reference** | **CARB1** | **CARB2** | **CARB3** | **CARB4** | **CARB5** | **CARB6** |
| Std-F | 97% | 99% | 101% | 99% | 100% | 99% | 99% |
| Std-E | 98% | 100% | 101 % | 99% | 99% | 99% | 99% |
| Std-D | 98% | 100% | 101% | 99% | 99% | 100% | 99% |
| Std-C | 97% | 100% | 101% | 99% | 100% | 99% | 98% |
| Std-B | 98% | 99% | 101% | 99% | 99% | 99% | 98% |
| Std-A | 98% | 99% | 100% | 99% | 99% | 99% | 98% |

All thiourea derivatives shown in Table 5 appear to have a comparable stabilizing effect as thiourea itself.

## Claims

1. A composition containing
a) a purified conjugate comprising two moieties covalently linked by a thiourea linker and
b) thiourea or a derivative thereof in free form,
wherein said thiourea derivative is selected from Dithiobiurea, alkylated thiourea having one, two, three or all four hydrogen atoms of thiourea substituted with alkyl of six or less carbon atoms, thiourea having one or both the nitrogens encompass acetyl or C1-C6 alkenyl and thiourea having an alkyl or alkylen bridge between the two nitrogen atoms of two or three C-atoms.

2. The composition of claim 1, wherein in said conjugate at least a first moiety that is capable of binding to a binding partner is comprised.

3. The composition of claim 2, wherein said moiety is capable of binding to an antibody.

4. The composition of any of claims 1 to 3, wherein in said conjugate at least a second moiety is comprised that is labeling group.

5. The composition of any of claims 1 to 4, wherein the thiourea in free form is present in an at least equimolar concentration as compared to the thiourea linker comprised in the conjugate.

6. A method for stabilizing a conjugate comprising two moieties covalently linked by a thiourea linker, the method comprising
a) providing the conjugate
b) adding thereto thiourea or a derivative thereof in free form and
c) thereby stabilizing the conjugate
wherein wherein said thiourea derivative is selected from Dithiobiurea, alkylated thiourea having one, two, three or all four hydrogen atoms of thiourea substituted with alkyl of six or less carbon atoms, thiourea having one or both the nitrogens encompass acetyl or C1-C6 alkenyl and thiourea having an alkyl or alkylen bridge between the two nitrogen atoms of two or three C-atoms.

7. The method of claim 6, wherein said conjugate comprises at least a first moiety that is capable of binding to a binding partner.

8. The method of claim 6 or 7, wherein in said conjugate at least a second moiety is comprised that is a labeling group.

9. The method of any of claims 6 to 8, wherein the thiourea in free form is added to result in a final concentration that is at least an equimolar concentration as compared to the thiourea linker comprised in the conjugate.

10. Use of thiourea or a derivative thereof as a stabilizer for a conjugate comprising two moieties covalently linked by a thiourea linker,
wherein said thiourea derivative is selected from Dithiobiurea, alkylated thiourea having one, two, three or all four hydrogen atoms of thiourea substituted with alkyl of six or less carbon atoms, thiourea having one or both the nitrogens encompass acetyl or C1-C6 alkenyl and thiourea having an alkyl or alkylen bridge between the two nitrogen atoms of two or three C-atoms.

## Patentansprüche

1. Zusammensetzung, umfassend:
a) ein gereinigtes Konjugat, umfassend zwei kovalent über einen Thioharnstoff-Linker verknüpfte Einheiten, und
b) Thioharnstoff oder ein Derivat davon in freier Form,
wobei das Thioharnstoff-Derivat ausgewählt ist aus Dithiobiharnstoff, alkyliertem Thioharnstoff, bei dem ein, zwei, drei oder alle vier Wasserstoffatome von Thioharnstoff substituiert sind mit Alkyl mit sechs oder weniger Kohlenstoffatomen, Thioharnstoff, bei dem ein oder beide Stickstoffatome Acetyl oder C1-C6-Alkenyl umfassen, und Thioharnstoff mit einer Alkyl- oder Alkylenbrücke zwischen den beiden Stickstoffatomen von zwei oder drei C-Atomen.

2. Zusammensetzung nach Anspruch 1, wobei das Konjugat mindestens eine Einheit umfasst, die an einen Bindungspartner binden kann.

3. Zusammensetzung nach Anspruch 2, wobei die Einheit an einen Antikörper binden kann.

4. Zusammensetzung nach einem der Ansprüche 1 bis 3, wobei das Konjugat mindestens eine zweite Einheit umfasst, die eine Markierungsgruppe ist.

5. Zusammensetzung nach einem der Ansprüche 1 bis 4, wobei der Thioharnstoff in freier Form in mindestens einer äquimolaren Konzentration zugegen ist, im Vergleich zu dem Thioharnstoff-Linker, der in dem Konjugat zugegen ist.

6. Verfahren zum Stabilisieren eines Konjugates, das zwei kovalent über einen Thioharnstoff-Linker verknüpfte Einheiten umfasst, wobei das Verfahren umfasst:
a) Bereitstellen des Konjugates
b) Hinzufügen von Thioharnstoff oder eines Derivat davon in freier Form und
c) **dadurch** Stabilisieren des Konjugates
wobei das Thioharnstoff-Derivat ausgewählt ist aus Dithiobiharnstoff, alkyliertem Thioharnstoff, bei dem ein, zwei, drei oder alle vier Wasserstoffatome von Thioharnstoff substituiert sind mit Alkyl mit sechs oder weniger Kohlenstoffatomen, Thioharnstoff, bei dem ein oder beide Stickstoffatome Acetyl oder C1-C6-Alkenyl umfassen, und Thioharnstoff mit einer Alkyl- oder Alkylenbrücke zwischen den beiden Stickstoffatomen von zwei oder drei C-Atomen.

7. Verfahren nach Anspruch 6, wobei das Konjugat mindestens eine erste Einheit umfasst, die an einen Bindungspartner binden kann.

8. Verfahren nach Anspruch 6 oder 7, wobei das Konjugat mindestens eine zweite Einheit umfasst, die eine Markierungsgruppe ist.

9. Verfahren nach einem der Ansprüche 6 bis 8, wobei der Thioharnstoff in freier Form zugegeben wird, so dass eine Endkonzentration erhalten wird, die mindestens eine äquimolare Konzentration ist, im Vergleich zu dem Thioharnstoff-Linker, der in dem Konjugat zugegen ist.

10. Verwendung von Thioharnstoff oder eines Derivates davon als Stabilisator für ein Konjugat, das zwei kovalent über einen Thioharnstofflinker verknüpfte Einheiten umfasst,
wobei das Thioharnstoff-Derivat ausgewählt ist aus Dithiobiharnstoff, alkyliertem Thioharnstoff, bei dem ein, zwei, drei oder alle vier Wasserstoffatome von Thioharnstoff substituiert sind mit Alkyl mit sechs oder weniger Kohlenstoffatomen, Thioharnstoff, bei dem ein oder beide Stickstoffatome Acetyl oder C1-C6-Alkenyl umfassen, und Thioharnstoff mit einer Alkyl- oder Alkylenbrücke zwischen den beiden Stickstoffatomen von zwei oder drei C-Atomen.

## Revendications

1. Composition contenant :
a) un conjugué purifié comprenant deux fractions liées de manière covalente par un lieur à base de thio-urée ; et
b) une thio-urée ou un de ses dérivés sous forme libre,
dans laquelle ledit dérivé de thio-urée est choisi parmi la dithiobiurée, de la thio-urée alkylée dont un, deux, trois ou l'ensemble des quatre atomes d'hydrogène de la thio-urée est/sont substitué(s) avec un groupe alkyle contenant six atomes de carbone ou moins, une thio-urée dont un atome d'azote ou les deux englobent un groupe acétyle ou un groupe alcényle en C₁-C₆ et une thio-urée possédant un pont alkyle ou alkylène entre les deux atomes d'azote de deux ou trois atomes de carbone.

2. Composition selon la revendication 1, dans laquelle ledit conjugué comprend au moins une première fraction qui est capable de se lier à un partenaire de liaison.

3. Composition selon la revendication 2, dans laquelle ladite fraction est capable de se lier à un anticorps.

4. Composition selon l'une quelconque des revendications 1 à 3, dans laquelle ledit conjugué comprend au moins une deuxième fraction qui représente un groupe de marquage.

5. Composition selon l'une quelconque des revendications 1 à 4, dans laquelle la thio-urée sous forme libre est présente en une concentration au moins équimolaire par rapport au lieur à base de thio-urée compris dans le conjugué.

6. Procédé de stabilisation d'un conjugué comprenant deux fractions liées de manière covalente via un lieur à base de thio-urée, le procédé comprenant :
a) la fourniture du conjugué ;
b) l'addition à ce dernier d'une thio-urée ou d'un de ses dérivés sous forme libre ; et
c) la stabilisation qui s'ensuit du conjugué ;
dans lequel ledit dérivé de thio-urée est choisi parmi la dithiobiurée, de la thio-urée alkylée dont un, deux, trois ou l'ensemble des quatre atomes d'hydrogène de la thio-urée est/sont substitué(s) avec un groupe alkyle contenant six atomes de carbone ou moins, une thio-urée dont un atome d'azote ou les deux englobent un groupe acétyle ou un groupe alcényle en C₁-C₆ et une thio-urée possédant un pont alkyle ou alkylène entre les deux atomes d'azote de deux ou trois atomes de carbone.

7. Procédé selon la revendication 6, dans lequel ledit conjugué comprend au moins une première fraction qui est capable de se lier à un partenaire de liaison.

8. Procédé selon la revendication 6 ou 7, dans lequel ledit conjugué comprend au moins une deuxième fraction qui représente un groupe de marquage.

9. Procédé selon l'une quelconque des revendications 6 à 8, dans laquelle la thio-urée sous forme libre est ajoutée afin d'obtenir une concentration finale qui représente une concentration au moins équimolaire par rapport au lieur à base de thio-urée compris dans le conjugué.

10. Utilisation d'une thio-urée ou d'un de ses dérivés à titre de stabilisateur pour un conjugué comprenant deux fractions liées de manière covalente via un lieur à base de thio-urée,
dans laquelle ledit dérivé de thio-urée est choisi parmi la dithiobiurée, de la thio-urée alkylée dont un, deux, trois ou l'ensemble des quatre atomes d'hydrogène de la thio-urée est/sont substitué(s) avec un groupe alkyle contenant six atomes de carbone ou moins, une thio-urée dont un atome d'azote ou les deux englobent un groupe acétyle ou un groupe alcényle en C₁-C₆ et une thio-urée possédant un pont alkyle ou alkylène entre les deux atomes d'azote de deux ou trois atomes de carbone.
